(19) Europäisches Patentamt
European Patent Office
Office européen des brevets

(11) Numéro de publication : **0 362 042 B1**

# (12) FASCICULE DE BREVET EUROPEEN

(45) Date de publication du fascicule du brevet : **23.06.93 Bulletin 93/25**

(51) Int. Cl.$^5$ : **C12Q 1/68,** C12Q 1/66

(21) Numéro de dépôt : **89402618.6**

(22) Date de dépôt : **25.09.89**

(54) **Procédé d'analyse d'une séquence spécifique d'ADN ou d'ARN, réactifs et nécessaires pour sa mise en oeuvre.**

(30) Priorité : **26.09.88 FR 8812537**

(43) Date de publication de la demande : **04.04.90 Bulletin 90/14**

(45) Mention de la délivrance du brevet : **23.06.93 Bulletin 93/25**

(84) Etats contractants désignés : **AT BE CH DE ES FR GB IT LI LU NL SE**

(56) Documents cités :
**EP-A- 0 238 332**
**WO-A-87/00198**
**CHEMICAL ABSTRACTS, vol. 104, no. 23, 9 juin 1986, page 408, résumé no. 203487c, Columbus, Ohio, US;**
**NATURE, vol. 331, 4 février 1988, pages 461-462; H.A. ERLICH et al.: "Specific DNA amplification"**

(73) Titulaire : **INSTITUT NATIONAL DE LA SANTE ET DE LA RECHERCHE MEDICALE (INSERM)**
**101, rue de Tolbiac**
**F-75654 Paris Cédex 13 (FR)**

(72) Inventeur : **Nicolas, Jean-Claude**
**Le Phaeton Avenue du P. Saulas**
**F-34000 Montpellier (FR)**
Inventeur : **Balaguer, Patrick/ Les Escaliers**
**Esc. 9 Appt 89 2bis rue des Tourterelles**
**F-34090 Montpellier (FR)**
Inventeur : **Terouanne, Béatrice**
**1 rue Henri**
**F-34000 Montpellier (FR)**
Inventeur : **Boussioux, Anne-Marie**
**Hameau des Guilhems 12 rue Arnaud de Lart**
**F-34090 Montpellier (FR)**

(74) Mandataire : **Ayache, Monique et al**
**Cabinet LEPEUDRY 52 avenue Daumesnil**
**F-75012 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 362 042 B1

## Description

L'invention concerne l'analyse, c'est-à-dire la détection et/ou le dosage d'ADNs, de fragments d'ADNs ou d'ARNs au moyen de systèmes non radioactifs.

Elle a plus particulièrement pour objet un procédé d'analyse d'ADNs, de fragments d'ADNs ou d'ARNs au niveau d'un support utilisant des réactions de bioluminescence, sans étape de séparation ni de lavage, ainsi que des réactifs et nécessaires pour essais ou "kits", pour la mise en oeuvre de ce procédé.

Au cours de ces dernières années, le développement des techniques d'hybridation moléculaire a révolutionné nos idées sur la structure du gène. La sensibilité et la facilité de mise en oeuvre des procédés d'hybridation ont permis de les appliquer non seulement aux recherches fondamentales mais aussi au diagnostic de maladies congénitales et à la détection d'une grande variété d'ADNs pathogènes viraux et microbiens (références 1 et 2).

A l'heure actuelle, l'hybridation moléculaire est surtout mise en évidence à l'aide de sondes marquées au $^{32}P$, et l'utilisation des sondes non radioactives dites sondes "froides" à "marqueur unique" (voir par exemple référence 3 à 5) est encore très peu développée, peut-être en raison du manque relatif de fiabilité des systèmes mis au point jusqu'à présent.

Il apparaît donc clairement que cette technique ne pourra se développer de façon importante dans les laboratoires d'analyses cliniques que si l'on dispose de systèmes fiables, stables (dans le temps) et à temps de réponse "court", pour remplacer les sondes radioactives de courte vie et dont la détection peut demander plusieurs jours, avec tous les inconvénients bien connus liés à l'utilisation d'éléments radioactifs.

De plus, des techniques récentes utilisant des réactions d'amplification enzymatique de la cible (références 6 et 7) produisent, à partir de quelques molécules de cibles ADN, une quantité relativement importante de copies. Cette amplification permet de résoudre le problème de la sensibilité et de ce fait il est nécessaire de disposer de techniques de détection rapides et faciles à automatiser.

Pour remédier aux inconvénients, tels que manque de fiabilité, des sondes à marqueur unique, des inventeurs ont eu l'idée de recourir à des réactions d'hybridation dites de type sandwich, par analogie aux réactions connues sous cette dénomination dans le cas de dosages immunologiques.

La technique sandwich d'identification d'un ADN ou d'un ARN par hydridation repose de façon générale sur le principe suivant : le milieu dans lequel est supposé se trouver l'ADN ou l'ARN à identifier est mis en contact successivement ou simultanément avec deux oligonucléotides "modifiés" ne présentant pas entre eux de séquence complémentaire, mais complémentaires chacun d'une séquence de l'ADN ou de l'ARN à identifier.

L'un de ces oligonucléotides ("sonde de fixation") est fixé sur un support (référence 8) ou modifié par un substituant choisi de telle sorte qu'il permette de fixer directement ou indirectement l'oligonucléotide sur un support, lui-même modifié de façon adéquate. Ainsi, le substitutant sur la "sonde de fixation" peut être par exemple la biotine et le support peut porter des groupements avidine (référence 9), ou encore le substituant peut être un haptène, notamment la fluorescéine, et le support peut porter des anticorps spécifiques de cet haptène, par exemple des anticorps antifluorescéine (référence 10).

L'autre oligonucléotide ("sonde marquée" ou "reporter") porte un "marqueur". Ce marqueur peut être radioactif (références 8 et 10) ou non radioactif (sonde froide). Dans ce cas, le marqueur est de préférence optiquement détectable, directement (par exemple dans le cas d'un "fluorophore") ou indirectement, par exemple par action d'une "enzyme marqueuse" sur un substrat (références 9 et 10).

L'hybridation peut se faire en milieu solide-liquide (références 8 et 9), ou en milieu liquide, la fixation sur le support n'ayant lieu qu'ultérieurement (référence 10).

Dans tous les cas des lavages sont nécessaires afin d'éliminer notamment les excès de réactifs.

On a maintenant trouvé qu'il était possible de détecter, et dans certains cas de doser, une séquence spécifique d'ADN ou d'ARN, sans étape de séparation ou de lavage, par réaction de bioluminescence, avec obtention d'un signal stable, de manière rapide et fiable, en associant essentiellement un système de capture dont un élément est coimmobilisé sur un support présentant une surface poreuse et/ou fibreuse avec les enzymes d'un système de bioluminescence, et un système de marquage utilisant une enzyme telle qu'une deshydrogénase.

L'invention faisant l'objet de la présente demande est basée sur cette constatation surprenante et concerne, selon l'un de ses aspects, un procédé d'analyse d'une séquence spécifique d'ADN ou d'ARN, caractérisé en ce qu'il comprend les étapes consistant, en utilisant :

a) un support présentant une surface poreuse et/ou fibreuse sur laquelle sont immobilisés d'une part, les enzymes luciférase et oxydoréductase d'un système de bioluminescence et d'autre part, un substituant capable de fixer, directement ou indirectement, par l'intermédiaire d'au moins une réaction d'affinité ou par hybridation spécifique, une séquence nucléotidique,

b) un conjugué d'enzyme comprenant une enzyme capable de produire un composé nucléotide intermédiaire, utilisable par l'oxydoréductase du système de bioluminescence, pour conduire à l'émission d'un signal de bioluminescence, et d'une molécule capable de se lier par affinité avec un marqueur de séquence nucléotidique ou de s'hybrider avec une telle séquence, et

c) une solution aqueuse comprenant les substrats de l'enzyme du conjugué, les constituants du système de bioluminescence autres que les enzymes et le composé nucléotide intermédiaire, ainsi que les constituants d'un système de destruction du composé nucléotide intermédiaire produit par l'enzyme dudit conjugué non reliée au support, à

1°- soit mettre un échantillon contenant l'ADN ou l'ARN à analyser, éventuellement amplifié, au moins doublement marqué par marquage chimique ou enzymatique avant l'analyse, par marquage synthétique au cours de l'amplification ou par hybridation avec une ou plusieurs sondes spécifiques, en contact, simultanément ou successivement, avec un support a) et un conjugué b) appropriés,

- soit mettre en échantillon contenant l'ADN ou l'ARN à analyser, de préférence amplifié, non marqué, en présence de séquences homologues d'ADN ou d'ARN, de préférence amplifiées, doublement marquées et soumettre l'ensemble à dénaturation puis à réhybridation, et mettre le mélange obtenu en contact, simultanément ou successivement, avec un support a) et un conjugué b) appropriés,
- soit mettre en contact, simultanément ou successivement, d'une part un échantillon contenant l'ADN ou l'ARN à analyser, de préférence amplifié, avec un support a) portant une séquence nucléotidique complémentaire d'une partie dudit ADN ou ARN, cette séquence pouvant être fixée sur le support au cours de l'analyse, par réaction d'affinité spécifique entre un substituant du support et un substituant porté par un oligonucléotide présentant une affinité spécifique pour ledit substituant du support et, d'autre part un conjugué d'enzyme b) dans lequel l'enzyme est conjuguée à une séquence nucléotidique complémentaire d'une autre partie dudit ADN ou ARN et non complémentaire de la séquence nucléotidique portée par la support ;

2° mettre l'ensemble obtenu sous 1° en présence d'un excès de la solution c) ;

3° laisser reposer jusqu'à obtention d'un signal stable; puis

4° mesurer la lumière émise; et éventuellement

5° déterminer, à l'aide d'un système d'étalonnage, la quantité de séquence à doser dans l'échantillon étudié, à partir de la mesure effectuée sous 4°.

Il convient ici de rappeler que le phénomène de bioluminescence résulte de l'action de l'oxygène catalysée par une enzyme de type luciférase, selon un schéma réactionnel plus ou moins complexe, sur un composé à l'état réduit, notamment nucléotidique tel que le NADH ou le NADPH. La luciférase la plus couramment utilisée est une luciférase d'origine bactérienne ou luciférase de Beneckea Harveyi.

Cette luciférase, en présence de NADH (nicotinamide dinucléotide sous forme réduite), d'une oxydoréductase spécifique provenant également de B. Harveyi et d'un aldéhyde à longue chaîne RCHO, tel que par exemple le décanal, produit de la lumière selon les réactions suivantes :

$$NADH + H^+ + FMN \xrightarrow{\text{oxydoréductase}} NAD^+ + FMNH_2 \text{ et}$$

$$FMNH_2 + RCHO + O_2 \xrightarrow{\text{luciférase}} FMN + RCOOH + H_2O + h\nu$$

Le même processus peut s'appliquer au NADPH.

Le NADH [ou NADPH] ainsi mis en évidence peut être produit dans le milieu réactionnel par action d'une deshydrogénase sur son substrat spécifique et sur le $NAD^+$ [ou $NADP^+$].

Cette deshydrogénase peut être par exemple la glucose-6-phosphate deshydrogénase (G-6-PDH) dont le substrat spécifique est le glucose-6-phosphate (G-6-P) ou la malate deshydrogénase dont le substrat spécifique est le malate. Les réactions mises en jeu sont alors respectivement :

$$G\text{-}6\text{-}P + NAD^+ [\text{ou } NADP^+] \xrightarrow{G\text{-}6\text{-}PDH} \text{acide 6-phosphogluconique} + NADH\ [\text{ou } NADPH] + H^+ \text{ et}$$

$$\text{malate} + NAD^+ [\text{ou } NADP^+] \xrightarrow[\text{deshydrogénase}]{\text{malate}} \text{oxalacetate} + NADH\ [\text{ou } NADPH] + H^+$$

On pourrait également utiliser un système qui produise dans le milieu, en tant que composé nucléotide intermédiaire, du FMN à partir de $FMNH_2$.

Il s'est toutefois révélé avantageux d'avoir, comme composé nucléotide intermédiaire, du NADH ou du NADPH.

Le support au voisinage duquel se produisent les différentes réactions impliquées dans l'analyse présente une surface poreuse et/ou fibreuse. Il est avantageusement constitué d'un polymère osidique tel que l'agarose ou un agarose modifié, d'acrylamide, d'un copolymère acrylique, de verre poreux, d'une matière fibreuse, notamment.

Il peut se présenter sous différentes formes, notamment sous forme de film. De préférence toutefois, il se présente sous forme de microbilles ayant jusqu'à environ 1 mm, de préférence environ 100 µm de diamètre.

Les matériaux à base d'agarose modifié, se présentant sous forme de microbilles, commercialisés sous la dénomination de Sepharose® par la Société Pharmacia, constituent des supports avantageux pour la mise en oeuvre du procédé selon l'invention.

Dans le cas où on utilise des microbilles, les différents éléments portés sur le support peuvent être fixés sur des microbilles différentes, à condition toutefois que ces différents éléments soient dans la proximité immédiate les uns des autres grâce à un mélange intime.

Dans ce cas, bien que cela ne présente pas a priori un grand intérêt, on peut prévoir d'introduire les microbilles portant les enzymes du système de bioluminescence après avoir effectué la réaction d'hybridation éventuelle et avant l'introduction des différents substrats.

Selon un mode préféré de réalisation de l'invention :

- l'enzyme du conjugué b) est la glucose-6-phosphate deshydrogénase et son substrat est le glucose-6-phosphate qui doit être présent dans la solution ;
- le composé nucléotide intermédiaire est le NADH qui est produit dans le milieu à partir de NAD, également présent dans ce milieu ;
- la luciférase est la luciférase de B. Harveyi qui est fixée sur le support a), notamment à base d'agarose, en présence de l'oxydoréductase correspondante ; le système de bioluminescence doit donc, d'après ce qui a été vu plus haut, comprendre du FMN et un aldéhyde à longue chaîne tel que le décanal.

Par ailleurs, le composé nucléotide intermédiaire produit au niveau du support a) par l'enzyme du conjugué, notamment une deshydrogénase, alimente directement les réactions de bioluminescence. Le même composé produit dans la solution aqueuse par la même enzyme non reliée au support est en général suffisamment éloigné des enzymes du système de bioluminescence pour ne pas interférer avec le dosage, ou n'interférer que d'une façon négligeable. Toutefois, il convient d'introduire dans le milieu un système, de préférence enzymatique soluble, capable de transformer le composé nucléotide intermédiaire présent dans la solution en un composé non susceptible de donner lieu à l'émission de photons en présence du système de bioluminescence, sans modifier de façon importante le composé nucléotide intermédiaire formé au niveau du support. A titre illustratif, on peut indiquer par exemple que lorsque le composé nucléotide intermédiaire est du NADH, on peut le réoxyder dans la solution par un système enzymatique soluble tel que par exemple le système lactate deshydrogénase (LDH)-pyruvate, la réaction qui intervient est la suivante:

$$NADH + \text{pyruvate} \xrightarrow{LDH} NAD + \text{lactate}$$

En conséquence, le milieu dans lequel on effectue le dosage contient les constituants d'un système de destruction du composé nucléotide intermédiaire, par exemple, dans le cas indiqué ci-dessus, de la LDH et du pyruvate.

Les proportions relatives des différents constituants du système de dosage peuvent être déduites aisément

des exemples qui suivent. En tout état de cause, il convient que l'excès de marqueurs de détection ne soit pas supérieur à environ 100 fois la quantité de cible.

Selon un mode de réalisation du procédé de l'invention, à l'étape 1°, la séquence à analyser est substituée doublement, éventuellement au cours de l'analyse, par un premier substituant capable de se lier, directement ou indirectement, au support a) par une réaction d'affinité et par un second substituant capable de se lier au conjugué b) par une réaction d'affinité.

La double substitution de la séquence à analyser peut se faire par tout moyen connu tel que notamment "marquage chimique" ou "marquage enzymatique" de la séquence elle-même, ou encore hybridation avec une sonde ADN ou ARN doublement substituée ou marquée avec deux sondes ADN ou ARN, non complémentaires l'une de l'autre, portant chacune un seul substituant, les substituants de la sonde unique doublement marquée, d'une part, et des deux sondes à un seul substituant, d'autre part, étant différents.

Le "second substituant" peut être choisi notamment parmi la biotine, la photobiotine, l'avidine, la streptavidine, un haptène tel qu'un groupe sulfone, acétoxy acétyl amino fluorène (AAF), fluorescéine ou dinitrophénol (DNP), notamment.

Par exemple lorsque le "second substituant" est la biotine ou la photobiotine, le conjugué b) est avantageusement constitué de l'enzyme, par exemple une deshydrogénase, capable de produire un composé nucléotide intermédiaire, et d'avidine ou de streptavidine.

Au contraire, lorsque le "second substituant" est l'avidine ou la streptavidine, l'enzyme du conjugué b) est avantageusement conjuguée avec la biotine ou la photobiotine.

Par ailleurs, lorsque le "second substituant" est un groupe sulfone, AAF, fluorescéine ou DNP, l'enzyme du conjugué b) est avantageusement liée à des anticorps anti-ADN sulfone, anti-AAF, anti-fluorescéine, ou anti-DNP, respectivement.

Il va de soi que d'autres systèmes, notamment plus complexes, faisant intervenir des réactions d'affinité spécifique pourraient être imaginés par l'homme du métier sans sortir du cadre de la présente invention.

Le "premier substituant" a pour rôle d'amener la séquence d'ADN ou d'ARN doublement marquée, notamment sous forme hybridée, au voisinage immédiat du support a) afin d'y "confiner" les différentes réactions mises en jeu, à savoir notamment :

- la production par l'enzyme liée au premier substituant, d'une quantité de nucléotide intermédiaire réduit, proportionnelle à la quantité de la séquence d'ADN ou d'ARN recherchée, et
- la réaction subséquente de bioluminescence dépendante de cette quantité de nucléotide intermédiaire.

Le "premier substituant" peut également être choisi parmi la biotine, la photobiotine, l'avidine et la streptavidine, à condition qu'il n'y ait pas de risque de réaction croisée avec le "second substituant" ou le composé pour lequel il a une affinité spécifique, présent dans le milieu.

Avantageusement, lorsque le "premier substituant" est la biotine ou la photobiotine, le support a) porte des groupes avidine ou streptavidine et lorsque le "premier substituant" est l'avidine ou la streptavidine, le support a) porte des groupes biotine ou photobiotine. Toutefois, des systèmes plus complexes faisant par exemple intervenir, outre l'affinité particulière de type avidine-biotine, par exemple des réactions de type immunologique, peuvent être imaginés et utilisés.

Lorsque le "premier substituant" est un haptène tel qu'un groupe fluorescéine, DNP, AAF ou sulfone par exemple, la fixation de la séquence d'ADN ou d'ARN doublement substituée sur le support peut se faire par l'intermédiaire d'anticorps anti-haptène spécifiques (anticorps anti-fluorescéine etc., selon le cas).

Toutefois, selon un mode avantageux de réalisation de l'invention, la fixation a lieu au moyen d'une double réaction de type immunologique, la première faisant intervenir des anticorps spécifiques de l'haptène, présents dans le milieu et la seconde faisant intervenir la réaction de ces anticorps spécifiques avec la protéine A ou un anticorps anti-$\gamma$-globulines fixé(e) sur le support. Il va de soi que dans cette seconde hypothèse, le système "second substituant"/ enzyme du conjugué ne doit pas faire intervenir d'anticorps susceptibles de se lier avec la protéine A ou l'"anticorps anti-$\gamma$-globulines.

Le procédé de type "sandwich", à double substitution, décrit ci-dessus est applicable notamment :

- à la détection et au dosage d'une séquence d'ADN (ou d'ARN) "doublement marquée" par synthèse, c'est-à-dire portant des substituants différents introduits par des techniques de synthèse d'ADN ou d'ARN modifié ;
- à la détection et au dosage d'une séquence d'ADN (ou d'ARN) double brin, formée par hybridation de deux séquences d'ADN (ou d'ARN) portant chacune un seul "marqueur" ou substituant ; cette méthode peut être utilisée notamment lorsque la séquence d'ADN (ou d'ARN) cible peut être modifiée chimiquement ; la capture de cette séquence d'ADN (ou d'ARN) cible est alors réalisée au moyen d'une séquence d'ADN (ou d'ARN) complémentaire ou sonde "marquée" par un autre substituant ;
- à la détection et au dosage d'une séquence nucléotidique particulière, ou cible, après hybridation au moins partielle de celle-ci avec deux séquences complémentaires chacune d'une partie de la cible mais

non complémentaires entre elles, et portant l'une le "premier substituant" et l'autre le "second substituant".

L'application de ce procédé de type "sandwich" est particulièrement intéressante en combinaison avec la technique d'amplification de l'ADN (référence 7). Selon cette technique, deux oligonucléotides amorces ou "primers" "flanquent" le segment d'ADN à amplifier en s'hybridant aux brins opposés, après dénaturation. L'extension de l'amorce par polymérisation se fait au moyen d'une polymérase, de préférence une polymérase résistante à la chaleur telle que la polymérase Taq décrite à la référence 7. Cette technique, comme il sera vu plus loin dans les exemples, peut avantageusement être appliquée à la mise en évidence d'un gène particulier. Après un certain nombre de cycles, on dispose d'un nombre de copies de la séquence recherchée suffisant pour permettre son identification, voire même son dosage, par le procédé selon l'invention.

Ainsi, selon un mode avantageux de réalisation, le procédé selon l'invention, sous sa forme de type "sandwich", comprend avant la mise en contact de l'hybride avec le support a), le conjugué b) et les réactifs de bioluminescence, une série de cycles de dénaturation et d'amplification de l'ADN ou de l'ARN, au moyen d'une polymérase.

Selon un autre mode de réalisation du procédé de l'invention utilisant une réaction d'hybridation de type "sandwich", le support a) porte une séquence nucléotidique capable de s'hybrider avec la séquence à analyser, complémentaire d'une partie de cette séquence et le conjugué b) est constitué d'une enzyme, notamment une deshydrogénase, et d'une séquence nucléotidique, non complémentaire de la séquence portée par le support a) et complémentaire d'une partie de la séquence à analyser, de préférence amplifiée.

Selon encore un autre mode de réalisation du procédé de l'invention utilisant une réaction d'hybridation de type "sandwich", la séquence à analyser, de préférence amplifiée, est marquée par un substituant présentant une affinité spécifique pour le substituant porté par le support a), et le conjugué b) est constitué d'une enzyme, notamment une deshydrogénase, et d'une séquence nucléotidique complémentaire d'une partie de la séquence à analyser.

Selon un autre mode de réalisation du procédé de l'invention faisant intervenir une réaction de type "sandwich", la séquence à analyser est multimarquée, de préférence par amplification, le support a) porte des substituants présentant une affinité spécifique pour le marqueur de ladite séquence et le conjugué b) comporte également un substituant présentant une affinité spécifique pour le marqueur de ladite séquence.

Selon un autre mode de réalisation, l'étape 1° du procédé de l'invention fait intervenir une réaction de compétition utilisant une séquence doublement marquée par des substituants différents ou deux oligonucléotides complémentaires marqués par des substituants différents, lesdits substituants différents présentant l'un, une affinité spécifique pour le substituant spécifique porté par le support a) et l'autre, une affinité spécifique pour la molécule spécifique du conjugué b).

Dans ce cas la séquence à analyser ou cible n'est pas marquée et l'ensemble des séquences nucléotidiques est soumis à dénaturation puis à une réhybridation. Lors de la mise en oeuvre de l'étape 4°, le signal lumineux diminue en fonction de la quantité de cible présente dans le milieu.

Les différents modes de réalisation de l'invention sont expliqués plus en détail dans les exemples non limitatifs qui suivent.

l'invention a également pour objet un réactif pour la mise en oeuvre du procédé défini ci-dessus.

Un tel réactif est caractérisé en ce qu'il se présente sous la forme d'un support présentant une surface poreuse et/ou fibreuse sur laquelle sont immobilisés d'une part, les enzymes luciférase et oxydoréductase d'un système de bioluminescence et d'autre part, un substituant capable de fixer, directement ou indirectement, par l'intermédiaire d'au moins une réaction d'affinité ou par hybridation spécifique, une séquence nucléotidique.

Ce support peut être par exemple la paroi d'un tube dans lequel sera effectuée la réaction, ou une "languette" que l'on plonge dans la solution aqueuse où est effectué le dosage. De préférence cependant, il se présente sous forme de microbilles, par exemple de Sepharose®. Ces microbilles peuvent avantageusement être agglomérées sous forme de comprimé enrobé, délitable dans l'eau, ou se présenter sous forme de suspension dans l'eau.

L'immobilisation du substituant capable de fixer par affinité ou hybridation une séquence nucléotidique et des enzymes sur le support est de préférence effectuée de façon covalente, sur la base des connaissances usuelles de l'homme du métier en cette matière. Une description plus précise d'une telle fixation est donnée dans les exemples qui suivent.

Selon un mode de réalisation, l'entité capable de fixer par affinité une séquence nucléotidique est un anticorps anti-$\gamma$-globulines, une protéine A ou l'avidine.

selon un autre mode de réalisation, l'entité capable de fixer par hybridation une séquence nucléotidique est un oligonucléotide complémentaire d'une partie de ladite séquence, ou sonde nucléique.

Selon un mode tout particulièrement préféré de réalisation, l'invention fournit un réactif, caractérisé en ce qu'il est constitué de microbilles d'agarose modifié portant, fixés à leur surface sur les mêmes microbilles ou

sur des microbilles différentes, un anticorps anti-γ-globulines, une protéine A, de l'avidine ou une sonde nucléique, une luciférase d'origine bactérienne et une oxydoréductase.

Cette oxydoréductase est avantageusement la FMN oxydoréductase.

L'invention a en outre pour objet un "nécessaire" ou "kit" pour la mise en oeuvre du procédé décrit ci-dessus.

Ce nécessaire comprend :

- le réactif selon l'invention décrit plus haut, à l'abri de la lumière, et
- un flacon contenant un conjugué de deshydrogénase et d'une molécule capable de se lier sélectivement, par affinité, avec un marquer de séquence nucléotidique ou de s'hybrider avec une telle séquence.

La molécule capable de se lier sélectivement par affinité peut être un anticorps, l'avidine, la biotine etc.

Selon un mode particulier de réalisation, le nécessaire selon l'invention comprend :

- un flacon contenant, à l'abri de la lumière, des microbilles d'agarose modifié, en suspension dans l'eau, ou sous forme de comprimé délitable dans l'eau, munies à leur surface d'un anticorps anti-γ-globulines ou d'une protéine A, d'une luciférase d'origine bactérienne et d'une oxydoréductase,
- un flacon contenant un conjugué de deshydrogénase et d'une molécule capable de se lier sélectivement, par affinité, à un marqueur de séquence nucléotidique ou de s'hybrider avec une telle séquence,
- un flacon contenant un anticorps spécifique d'un autre marqueur de séquence nucléotidique, et
- du tampon, du NAD ou du NADP, du FMN, un aldéhyde à longue chaîne, un système capable de détruire le NADH ou le NADPH en solution, ainsi que le substrat de la deshydrogénase.

Selon un autre mode particulier de réalisation, le nécessaire selon l'invention comprend :

- un flacon contenant, à l'abri de la lumière, des microbilles d'agarose modifié, en suspension dans l'eau, ou sous forme de comprimé délitable dans l'eau, munies à leur surface d'avidine ou de streptavidine, d'une luciférase d'origine bactérienne et d'une oxydoréductase,
- un flacon contenant un conjugué de deshydrogénase et d'une molécule capable de se lier sélectivement, par affinité, à un marqueur de séquence nucléotidique ou de s'hybrider avec une telle séquence, et
- du tampon, du NAD ou du NADP, du FMN, un aldéhyde à long chaîne, un système capable de détruire le NADH ou le NADPH en solution, ainsi que le substrat de la deshydrogénase.

Il va de soi que le "nécessaire" en question peut comprendre, selon le cas, d'autres constituants, notamment une ou plusieurs sondes.

La composition des différentes variantes du " nécessaire" selon l'invention peut se déduire aisément de la description qui précède et des exemples qui suivent.

Il va de soi que d'autres variantes du procédé selon l'invention, faisant intervenir les mêmes principes, peuvent être imaginées par l'homme du métier sur la base de ses connaissances usuelles en la matière, sans sortir du cadre de l'invention. Dans ce contexte, on peut par exemple prévoir que l'enzyme capable de produire un composé nucléotide intermédiaire soit fixée sur la séquence qui sera liée au support a), sans intervention d'un conjugué b).

## MATERIEL ET METHODES

### 1) Préparation des conjugués streptavidine, avidine ou anticorps/G6PDH

8 μl d'une solution d'anhydride S-acetyl mercapto succinique (SAMSA) $10^{-1}$M dans de l'acétonitrile, sont ajoutés à la protéine à coupler avec l'enzyme et placée dans de la glace (1 mg dans 300 μl de tampon phosphate 0,1 M, pH 7,4). Après 30 min. à 4°C, l'incubation est arrêtée par addition de 35 μl d'hydroxylamine 1M ($NH_2OH$) et 5 μl de dithiothréitol (DTE) ($10^{-1}$M). La protéine thiolée est séparée de l'excès de réactifs par chromatographie sur une colonne de substance dérivée du dextrane, commercialisée sous la dénomination Sephadex G-25 par la société Pharmacia et équilibrée avec du tampon $PO_4^{2-}$ 0,05 M, pH 6,4.

1,4 mg de G6PDH sont mis en solution dans 500 μl de ce même tampon, avec 50 μl de NADPH $10^{-2}$M, 50 μl de G6P 3X$10^{-1}$M, 20 μl d'EDTA $10^{-1}$M. 20 à 40 μl d'ester N-hydroxysuccimidique du maléimido cyclohexane carboxylate (SMCC) 5X$10^{-4}$M préparés dans du DMF sont ajoutés en deux fois au mélange. L'incubation dure 1 heure à température ambiante, puis l'excès de SMCC est éliminé par chromatographie sur une colonne de Sephadex G-25.

La protéine de liaison thiolée et la G6PDH modifiée sont incubées ensemble pendant 2 heures à température ambiante. Le mélange réactionnel est concentré sur une membrane filtrante, telle que celle commercialisée par la société AMICON sous la dénomination Amicon® 30, à un volume final de 200 μl. Les conjugués de la G6PDH sont séparés de l'enzyme libre par chromatographie.

2) Préparation des immunoadsorbants bioluminescents :

Les enzymes du système de bioluminescence utilisées, luciférase et NADH [ou NADPH] réductase-FMN spécifique, appelée ci-après FMN oxydoréductase d'origine bactérienne, sont préparées par culture de Beneckea Harveyi selon le procédé de A.H. Farghaly (référence 11) et purifiées selon la méthode de A. Gunsalus-Miguel et coll. (référence 12), telle que modifiée par T.O. Baldwin et coll. (référence 13).

Le système luciférase-oxydoréductase est coimmobilisé sur Sepharose® 4B avec un anticorps anti-γ-globulines, ou avec l'avidine ou la streptavidine.

Le Sepharose 4B activé au bromure de cyanogène est lavé par 200 ml de HCl $10^{-3}$M et mis en suspension dans 5 ml de pyrophosphate de potassium 0,1 M contenant 4 mg d'anticorps, d'avidine ou de streptavidine, 2,5 mg de luciférase et 2 UI/ml d'oxydoréductase. La solution est agitée toute une nuit à 4°C, puis le Sepharose est rincé par 200 ml de tampon pH 7,4 et conservé en présence de DTE $3X10^{-3}$M.

3) Détection des ADNs ou ARNs doublement marqués par bioluminescence

La solution d'ADN ou d'ARN est incubée 30 min. à 30°C avec la G6PDH couplée à l'anticorps, l'avidine ou la streptavidine (1 mUI) et le support de capture (3 mg) sur lequel sont immobilisées les enzymes de luminescence et la protéine permettant la liaison de l'ADN ou de l'ARN marqué (protéine A, anticorps, avidine par exemple). Après addition des substrats FMN $5X10^{-5}$M, décanal 5 $10^{-6}$M, NAD $10^{-3}$M, G6P $10^{-3}$M, pyruvate $10^{-3}$M, LDH 1 à 5 UI/ml en solution dans 1 ml de tampon pH 7,5 0,1 M, la luminescence est mesurée pendant 10 s.

4) Dosage d'ADNs doublement marqués par synthèse

a) On prépare un phage λ marqué par la biotine par réaction de nick translation en présence de biotine 11 dUTP (référence 14) ou par marquage chimique par la photobiotine (référence 15). Le phage est ensuite modifié par une réaction de sulfonation (référence 16).

b) On prépare un phage M13 modifié par réaction avec l'AAF (acétoxy acétyl amino fluorène) (référence 17) et la photobiotine.

Les ADNs ainsi obtenus sont capturés sur un support de Sepharose portant des groupes avidine ou des anticorps anti-ADN sulfone et les enzymes de bioluminescence.

Le dosage est réalisé dans un tampon 0,1 M Tris HCl pH 7,5 contenant 1 mUI de conjugué de la G6PDH et 2 mg du support mentionné ci-dessus, dans les conditions mentionnées en 3).

5) Détection et dosage d'ADNs formés par hybridation de deux ADNs simplement marqués.

a) Phage λ marqué par la biotine par réaction de nick translation ou en utilisant la photobiotine et phage λ marqué par une réaction de sulfonation. Les deux phages modifiés sont ensuite hybridés en solution à différentes concentrations et dans un tampon de force ionique élevée (NaCl 0,5 à 1 M). Un excès de l'ADN marqué par la biotine (50 ng) est utilisé pour réaliser l'hybridation avec l'ADN marqué par sulfonation. Les hybrides obtenus sont dosés par bioluminescence en utilisant un support sur lequel est immobilisé soit l'avidine soit un anticorps anti ADN sulfone.

b) Phage M13 simple brin modifié par l'AAF et porteur d'un insert de pBR322 et phage M13 marqué par la photobiotine et contenant un insert complémentaire de l'insert précédent. La mesure de luminescence est réalisée grâce à un support "bioluminescent" sur lequel a été immobilisée la protéine A permettant de lier l'anticorps anti-guanine-AAF.

Cette méthode est utilisable dans le cas où l'ADN cible peut être modifié chimiquement, par exemple par sulfonation. La capture de l'ADN cible est réalisée par une sonde marquée par une autre molécule, par exemple par un ADN biotinylé.

6) Dosage sandwich d'une cible

a) Le premier modèle concerne l'utilisation d'oligonucléotides. Il est constitué d'un poly A servant de cible et de deux oligonucléotides de 15 bases (oligo dT). Ces deux oligonucléotides sont modifiés de façon à introduire un groupement biotine ou un haptène comme la fluorescéine ou le DNP sur une extrémité. La modification peut être réalisée enzymatiquement ou chimiquement.

Marquage enzymatique

Les nucléotides dUTP ou dATP portant un résidu biotine ou $NH_2$ sur la base sont actuellement commercialisés. La fonction aminée permet de coupler des haptènes comme l'isothiocyanate de fluorescéine ou le fluorodinitrobenzène. Ces nucléotides ainsi fonctionnalisés sont ajoutés à l'extrémité 3′ de l'oligonucléotide grâce à l'action de la terminal transférase selon les conditions décrites à la référence 18 (2 à 4 bases peuvent être introduites par oligonucléotide).

Marquage chimique

Lors de la synthèse des oligonucléotides un groupement $NH_2$ peut être introduit en position 5' par réaction avec un amino éthyl ou hexyl phosphoramidite (référence 19). L'oligonucléotide aminé est ensuite couplé à la biotine ou à la fluorescéine par réaction avec un ester N-hydrosuccinimidique ou un dérivé de type isothiocyanate (référence 20). Le couplage à une enzyme peut également être réalisé en couplant l'ester N-hydroxysuccimidique du maléimido benzoate à l'oligonucléotide. L'enzyme, sur laquelle des résidus thiols ont été introduits, est incubée pendant plusieurs heures avec l'oligonucléotide modifié. Les produits de réaction sont séparés par chromatographie de type filtration sur gel.

b) Dans le deuxième modèle étudié, la cible à détecter est le pBR322 contenant un insert composé d'une séquence spécifique d'un gène. Les sondes de capture sont constituées par des phages M13 contenant le même insert. La sonde de détection est le pBR322 dans lequel cet insert est absent (plasmide pKTH). Les phages M13 reconnaissent les deux brins de l'insert tandis que le plasmide reconnaît le reste de la cible.

Les phages M13 sont marqués chimiquement par l'AAF tandis que le plasmide pKTH est biotinylé par nick translation.

Le système de capture est un Sepharose®-protéine A permettant de retenir un anticorps anti-AAF et donc de capturer les phages M13. La détection est assurée par le conjugué streptavidine-G6PDH qui reconnaît la biotine du plasmide pKTH.

c) Le troisième modèle utilisé par les inventeurs concerne l'analyse de la partie hypervariable du gène codant pour l'antigène humain d'histocompatibilité. Ce fragment d'ADN est obtenu par amplification enzymatique en utilisant deux oligonucléotides décrits dans la littérature (référence 21). Les oligonucléotides sont fonctionnalisés en position 5' par la biotine ou la fluorescéine. Le résultat de l'amplification est une molécule doublement marquée qui peut être dosée par bioluminescence. Dans ce modèle il est intéressant de connaître la nature de la séquence amplifiée par une analyse plus fine grâce à des oligonucléotides spécifiques. Les inventeurs ont donc synthétisé ces oligonucléotides sondes couplés à la fluorescéine, la biotine ou une enzyme et les hybrides correspondants ont été dosés par bioluminescence.

## RESULTATS

### EXEMPLE 1: Phage doublement marqué

Il est possible de détecter par le procédé selon l'invention toute molécule d'ADN sur laquelle deux marqueurs ont été fixés. Avec un phage λ doublement marqué par la biotine et par sulfonation, on constate que l'émission de lumière détectée par un luminomètre, par exemple celui commercialisé par la société LKB, est proportionnelle à la quantité d'ADN doublement marqué.

La limite de détection de l'ADN de phage λ est de 0,1 pg lorsqu'une base sur quarante est modifiée soit par la biotine soit par sulfonation.

### EXEMPLE 2 : ADN formé par hybridation de deux ADNs marqués

Les possibilités de cette méthode ont été étudiées en utilisant des sondes constituées d'inserts clonés dans M13. En utilisant comme système de capture un excès de phage biotinylé, il est possible de détecter par hybridation des quantités de M13 marqué par sulfonation, comprises entre 1 pg et 10 ng.

### EXEMPLE 3 : Dosage sandwich d'une cible

1er modèle : dosage d'une cible simple brin

Le premier exemple d'un dosage sandwich a été réalisé sur un modèle simple constitué par une cible simple brin, le poly A, et deux oligonucléotides dT15. Des quantités variables de poly A sont incubées 30 min. avec

3 femtomoles de chacun de deux oligonucléotides marqués respectivement par la biotine et par la fluorescéine dans 20 µl de tampon de force ionique élevée (NaCl 0,5 à 1 M). Après hybridation, de l'avidine-G6PDH (1 mUI) et un anticorps antifluorescéine (dilution de l'immunsérum 1/1000) sont ajoutés et le mélange est ensuite dilué et ajouté à du Sepharose® (3 mg) sur lequel ont été immobilisées les enzymes de luminescence et un anticorps anti-λ-globulines. Les substrats des enzymes (NAD, G6P, FMN, décanal) sont ajoutés et la luminescence mesurée en présence de LDH et de pyruvate pour détruire l'excès de NADH produit par l'enzyme non liée.

On obtient un signal proportionnel à la quantité de poly A. En utilisant 3 femtomoles de chaque sonde, il est possible de détecter 0,3 pg de poly A, soit 30 attomoles de séquence de reconnaissance ou cible. Le dosage peut être réalisé sans lavage, la suspension étant simplement diluée. Il a été constaté que des lavages successifs n'apportent qu'une faible amélioration de la sensibilité.

2ème modèle : dosage d'une cible double brin

Le sensibilité diminue considérablement lorsqu'on réalise une réaction sandwich pour doser une cible double brin. En utilisant deux sondes M13 marquées par l'AAF (capture) et un plasmide marqué par la biotine (détection) ce système ne permet de détecter que des quantités de plasmide cible supérieures à 50 à 100 pg. Cette faible sensibilité provient notamment des faibles rendements d'hybridation. Du fait de cette faible sensibilité, la méthode sandwich n'a pu être utilisée que pour détecter des cibles en quantités appréciables comme dans le cas de la réaction d'amplification par la polymérase.

3ème modèle : dosage d'une cible double brin avec amplification

Par amplification d'ADNs humains en utilisant comme amorces ou "primers" deux oligonucléotides marqués en position 5' par la biotine et la fluorescéine respectivement, il est possible d'obtenir suffisamment de cible doublement marquée pour réaliser son dosage sur 0,1 à 5 µl des 100 µl du milieu d'amplification. La sensibilité est très élevée car la cible est amplifiée d'un facteur supérieur à $10^6$.

Cette méthode de dosage a été appliquée à l'identification des porteurs d'une séquence particulière pouvant être contenue dans le gène codant pour la chaîne légère de l'antigène humain d'histocomptabilité.

Elle consiste à amplifier selon les conditions décrites à la référence 7 la partie du gène à analyser, puis une faible fraction, 1 à 5 % du milieu, est utilisée pour déterminer la quantité de séquence doublement marquée obtenue par amplification. 1 à 5 µl de milieu est incubé 30 min. avec 5 mUI de conjugué (avidine- ou anticorps-G6PDH), puis ajouté à 3 mg de support bioluminescent sur lequel est immobilisé un anticorps antifluorescéine ou de l'avidine. La luminescence est mesurée après addition des substrats et du système de destruction du NADH en excès (1UI de LDH + pyruvate).

L'analyse plus fine de la séquence est réalisée avec amplification, en utilisant une sonde plus spécifique. En utilisant différentes sondes et en étudiant le rapport des signaux obtenus, il est possible de définir la nature exacte de la séquence qui a été amplifiée.

L'exemple ci-dessous a été obtenu après amplification d'ADN humain avec des oligonucléotides spécifiques du gène codant pour une chaîne de l'antigène d'histocompatibilité.

Deux ADNs humains ont été amplifiés dans 100 µl grâce aux oligonucléotides "amorces" S1 et S2 qui permettent d'amplifier la partie du gène à étudier :

Biotine HN -- GATTTCGTGTGTACCAGTTTAAGGGC
(oligo biotine S1)
Fluorescéine S=C=N--CCACCTCGTAGTTGTGTCTGCA
(oligo FITC S2)
Note : FITC = isothiocyanate de fluorescéine.

Après amplification (30 cycles) on obtient une certaine quantité d'hybrides doublement marqués qui peuvent être dosés par bioluminescence. 1 µl du milieu d'amplification est incubé avec le conjugué couplé à l'enzyme puis ajouté au support sur lequel sont immobilisées l'avidine (ou l'anticorps anti-fluorescéine) et les enzymes de luminescence.

Après addition des substrats, la luminescence est mesurée.

Les résultats obtenus peuvent être comparés à ceux obtenus après amplification avec les sondes S2 et S3, S3 étant l'oligonucléotide spécifique de la séquence étudiée (ou hybridation avec S3 marquée par une enzyme). Il est nécessaire de savoir si la séquence S3 représentée ci-dessous est contenue dans le gène dans son intégralité.

Biotine-GGCGGCCTGTTGCCGAG (oligo biotine S3)

RESULTATS

| ADN | 1ère Mesure (S1-S2) | 2ème Mesure (S2-S3) |
|---|---|---|
| ADN 1 | 31400 | 7830 |
| ADN 2 | 30400 | 1910 |
| ADN NS* | 900 | 720 |

* ADN NS représente le "bruit de fond" de la méthode.

Ces résultats montrent :

- la spécificité de l'amplification obtenue sur l'ADN humain ;
- que l'ADN 2 ne contient pas exactement la séquence complémentaire de l'oligonucléotide S3. Bien que pouvant donner lieu à la 1ère réaction d'amplification, la valeur de la 1ère mesure étant équivalente à celle obtenue avec l'ADN 1, il donne un signal plus faible avec l'oligonucléotide S3 (2ème mesure). En effet cet ADN 2 contient bien le gène étudié mais il possède une séquence différente de l'ADN 1 (3 bases sur les 17 de l'oligonucléotide sont différentes).

La première mesure rend compte du rendement de l'amplification, la deuxième traduit la spécificité de l'hybridation de S3.

CONCLUSION

Les réactions de bioluminescence confinées sur un support poreux permettent de réaliser le dosage de fragments d'ADN ou d'ARN doublement marqués, notamment par des haptènes ou par la biotine. Cette méthode est utilisable pour le dosage de séquences spécifiques marquées chimiquement (sulfonation, AAF, etc.) mais aussi pour réaliser des dosages sandwichs d'une cible en utilisant deux oligonucléotides modifiés en 5', notamment par amplification enzymatique de la cible. Dans la mesure où l'excès de marqueurs de détection n'est pas supérieur à environ 100 fois la quantité de cible, la méthode ne nécessite pas d'étape de séparation.

Etude de l'analyse par bioluminescence de séquences d'ADN obtenues par la réaction d'amplification catalysée par une polymérase.

La technique de bioluminescence a été utilisée pour la détection ou le dosage de séquences d'ADN bi- ou monocaténaire, produites par une réaction d'amplification. Les analyses effectuées ont porté sur des séquences dont la concentration dans le milieu est comprise entre 100 à 10000 pg/ml.

Les modèles étudiés sont des séquences d'un virus de papillome humain ou des séquences du gène de l'antigène humain d'histocompatibilité (HLA) de différents ADNs humains incorporés ou non dans un plasmide (voir à ce sujet l'exemple 3, 3ème modèle, dans ce qui précède).

Les séquences étudiées ont été obtenues par réaction d'amplification catalysée par la polymérase de Thermus aquaticus. Deux oligonucléotides amorces définissent une séquence de 200 paires de bases. La réaction d'amplification est réalisée dans 100 µl (20 à 30 cycles) à partir de différentes quantités d'ADN cible [10 fg (femtogrammes) à 100 pg de plasmide correspondant à $10^3$ à $10^7$ molécules dans la prise d'essai, ou 1 µg d'ADN humain purifié].

Le protocole général utilisé pour l'amplification est le suivant :

dans un tube Eppendorf sont introduits :

- 10 µl de tampon d'amplification constitué de Tris HCl 670 mM, pH 8,8, $(NH_4)_2$ $SO_4$ 166 mM, $MgCl_2$ 67 mM, β-mercaptoéthanol 100mM, EDTA (acide éthylènediaminetétracétique) 67 mM, 1,7 g/l de gélatine, 0,1% de Triton ® X100 et 5% de DMSO (diméthylsulfoxyde);
- 10 µl de mélange de dCTP, dGTP, dATP et dTTP, chacun 2 mM;
- 1 µl d'ADN à une concentration de 1 ng/µl à 1µg/µl;

- qsp 100 µl d'eau; et
- 100 µl d'huile minérale.

Le tube est porté à ébullition pendant 5 min puis 2,5 UI de Taq polymérase(Proméga®)sont ajoutées et 20 à 30 cycles d'incubation sont réalisés (1 min à 95°C, 1 min à 56°C et 2 min à 72°C pour chaque cycle).

Le dosage par bioluminescence des séquences amplifiées peut être réalisé selon différents procédés :

- dosage sandwich
- dosage par compétition
- multimarquage spécifique de la séquence à étudier, qui sont étudiés et décrits dans les exemple 4 à 6 qui suivent.

Exemple 4 - Dosage sandwich.

Le dosage sandwich utilise un support sur lequel sont immobilisés les enzymes de luminescence et un système de capture de l'ADN à doser. Un oligonucléotide marqué par une enzyme permet, par hybridation spécifique, de doser l'ADN immobilisé.

Pour le dosage sandwich, la réaction d'amplification est réalisée en utilisant une quantité différente de chaque amorce (1 picomole et 50 picomoles, respectivement) afin de favoriser la formation d'ADN simple brin. L'amorce présente en défaut s'hybride plus difficilement, conduisant à une formation plus lente du brin complémentaire à celui que l'on veut doser (conditions "asymétriques").

**a) Phase oligonucléotide + oligonucléotide-enzyme.**

*Protocole*

Deux oligonucléotides reconnaissant deux zones adjacentes du même brin d'ADN sont utilisés. Un des oligonucléotides (A) est immobilisé sur un support comme le Sepharose®, conjointement avec les enzymes de luminescence (30 µg d'oligonucléotide, 5 mg de luciférase et 1 UI d'oxydoréductase pour 1 g de Sepharose® activé au (CNBr). L'immobilisation est réalisée en utilisant par exemple du Sepharose activé par le bromure de cyanogène et un oligonucléotide portant en position 5' un groupement aminé. La modification est réalisée au cours de la synthèse de l'oligonucléotide en utilisant un analogue de base modifié ou un produit de modification connu sous le nom d'Aminolink II ® (Applied Biosystem).Le deuxième oligonucléotide (B) porte également en 5' une fonction amine ou une fonction thiol permettant le couplage d'une enzyme. Le couplage est réalisé selon les méthodes connues décrites pour la préparation des conjugués protéiques (réaction d'un groupement maléimide porté par l'enzyme avec un résidu thiol porté par l'oligonucléotide). Le conjugué oligonucléotide-enzyme (G6PDH) est purifié sur colonne d'échangeur d'ions et concentré sur membrane filtrante (Amicon®30). Il est important que les deux oligonucléotides ne présentent pas de complémentarité afin d'éviter une liaison non spécifique.

Le dosage est réalisé en incubant simultanément à 37°C, de 1 à 3 heures, dans du tampon NaCl 0,15 M, Phosphate 0,1 M, pH 7,4 à 1‰ de BSA (albumine sérique bovine), en présence d'ADN de sperme de saumon à 10 µg/ml, 0,5 à 1 mg de Sepharose®-oligonucléotide, 0,1 mUI d'oligonucléotide-G6PDH et 1 à 20 µl de milieu d'amplification tel qu'obtenu comme décrit ci-dessus.

La quantité de G6PDH liée au support est mesurée après addition du réactif de luminescence (100 ml de tampon phosphate 0,1 M + 500 µl de FMN à 1 mg/ml + 500 µl de décanal à 2‰ + 500 µl de NAD $2.10^{-1}$ M + 500 µl de glucose-6P $6.10^{-1}$ M + 500 µl de pyruvate $6.10^{-1}$ M + 1 µl de LDH à 5 mg/ml).

*Résultats .*

Le tableau I qui suit montre les résultats obtenus (bioluminescence mesurée) avec différentes quantités de milieu d'amplification, en utilisant une séquence de virus de papillome humain dans un plasmide.

L'amplification a été réalisée, d'une part de façon symétrique, c'est-à-dire avec des amorces en quantités égales et, d'autre part de façon asymétrique, c'est-à-dire avec des quantités différentes des amorces, comme indiqué plus haut.

TABLEAU I

| volume (µl) | amplification symétrique | amplification asymétrique |
|---|---|---|
| 10 | | 8550 |
| 5 | 425 | 8472 |
| 1 | 380 | 4950 |
| 0,5 | 400 | 2450 |
| 0,1 | 246 | 977 |
| 0,025 | 198 | 390 |
| 0 | 186 | 190 |

Le tableau I montre que le signal obtenu est fonction de la quantité d'ADN produit par la réaction d'amplification. Il montre également que l'amplification asymétrique (qu conduit à la formation d'un seul brin complémentaire de la sonde) permet d'obtenir des signaux plus importants que l'amplification symétrique et une corrélation peut être établie (par exemple au moyen d'une courbe) entre la quantité d'ADN amplifié et l'intensité de luminescence mesurée.

Le tableau II qui suit montre les résultats obtenus avec des amplifications réalisées avec différentes quantités d'ADN cible (séquence de virus de papillome humain dans un plasmide).

TABLEAU II

| picogrammes ADN | amplification symétrique | amplification asymétrique |
|---|---|---|
| 100 | 2045 | 5280 |
| 1 | 2580 | 1900 |
| 0,1 | 1600 | 1223 |
| 0,01 | 563 | 214 |
| 0 | 320 | 105 |

On constate qu'une meilleure linéarité de la réponse en fonction de la quantité de cible est obtenue lorsque les amplifications sont réalisées en utilisant un des oligonucléotides amorces en quantité limitante afin de générer un ADN amplifié simple brin .

Dans ces conditions il es possible, après 30 cycles d'amplification, de détecter moins de 1000 molécules d'ADN viral et d'obtenir une réponse linéaire en fonction de la quantité d'ADN cible. La spécificité du dosage est apportée par la réaction d'amplification mais aussi par les deux réactions d'hybridation. En effet, bien que le protocole de dosage n'utilise pas d'étape de lavage, on obtient une bonne spécificité de la réaction d'hybridation car l'oligonucléotide marqué par l'enzyme est utilisé en concentration faible ($10^{-12}$ M). En présence de 1 µg d'ADN non spécifique, il est possible de détecter moins de 100 pg de la séquence amplifiée.

**b) phase streptavidine + oligonucléotide-enzyme.**

La sensibilité du dosage des séquences amplifiées peut être augmentée si la capture de la séquence amplifiées est réalisée directement par liaison sur une phase streptavidine .

*Protocole*

La réaction d'amplification est effectuée avec un oligonucléotide amorce biotinylé en 5', en excès par rapport à l'autre oligonucléotide amorce . Après amplification, 10 µl du milieu d'amplification sont incubés 1 à 3 heures avec l'oligonucléotide marqué par l'enzyme et 1 mg de Sepharose®-streptavidine-luciférase-FMN oxydoréductase (5 mg de streptavidine, 5 mg de luciférase et 1 UI de FMN oxydoréductase pour 1 g de Sepharose®). Les protocoles d'incubation et de mesure sont identiques à ceux précédemment décrits . La liaison au Sepharose® est dans ce cas réalisée par liaison de l'ADN biotinylé à la streptavidine, au lieu d'utiliser une réaction d'hybridation .

Les résultats obtenus sont rassemblés dans le tableau III qui suit.

TABLEAU III

| volume (µl) milieu d'amplification | amplification symétrique | amplification asymétrique |
|---|---|---|
| 5 | 1620 | 12510 |
| 1 | 1200 | 6200 |
| 0,5 | 820 | 3600 |
| 0,05 | 340 | 451 |
| 0 | 192 | 187 |

Le tableau III montre les résultats obtenus avec différents volumes d'amplification d'une séquence de gène d'un virus de papillome humain . La sensibilité obtenue est plus importante mais la spécificité est diminuée par la liaison au support de tous les fragments d'ADN biotinylés. D'autre part, afin d'éviter une saturation du support, la quantité de milieu d'amplification à doser doit être inférieure à 10 µl.

<u>Exemple 5 : dosage par compétition</u>

Le principe du dosage par compétition repose sur l'hybridation d'un ADN doublement marqué avec un ADN à doser non marqué. L'hybridation conduit à la disparition d'une molécule doublement marquée et donc à la disparition du signal de luminescence.

*Protocole*

L'ADN doublement marqué est préparé par amplification enzymatique en utilisant des oligonucléotides amorces marqués en position 5' par la biotine ou la fluorescéine. La séquence produite par amplification est marquée sur chaque brin. Après amplification, la molécule doublement marquée est purifiée par chromatographie ou par électrophorèse.

La molécule doublement marquée peut être également constituée par deux oligonucléotides complémentaires, marqués l'un par la biotine et l'autre par la fluorescéine.

Ces molécules doublement marquées, en présence de molécules homologues mais non marquées, sont soumises à une dénaturation par la chaleur et réhybridées pendant deux heures.

Le dosage de la molécule doublement marquée est réalisé par bioluminescence en utilisant un immunoadsorbant luminescent (5 mg d'anticorps anti-fluorescéine, 5 mg de luciférase et 1 UI de FMN oxydoréductase par g de Sepharose®). Les tampons d'hybridations et de mesure du signal lumineux sont les mêmes que précédemment . Le marquer enzymatique, à savoir la G6PDH, est couplé à la streptavidine. Cette protéine se lie à la biotine, tandis que la fluorescéine permet de retenir l'hybride sur l'immunoadsorbant.

*Résultats*

En l'absence de molécules homologues non marquées, la renaturation est pratiquement complète . Par

contre, en présence de séquences homologues d'ADN non marqué, la réhybridation conduit à la disparition de la molécule doublement marquée ainsi qu'en témoigne le tableau IV qui suit.

Dans ce cas, une séquence ADN, de 140 paires de bases (10 fmoles), marquée aux deux extrémités 5' par la biotine et la fluorescéine respectivement, est dénaturée et réhybridée en présence de différentes quantités de séquences amplifiées homologues, non marquées.

TABLEAU IV

| volume de milieu d'amplification (µl) | Signal (mvolts) |
|---|---|
| 2 | 156 |
| 1 | 199 |
| 0,5 | 228 |
| 0,25 | 311 |
| 0,125 | 404 |
| 0 | 1300 |
| témoin | 1350 |

<u>Etude de l'hybridation de deux oligonucléotides complémentaires en présence de séquences amplifiées de différentes longueurs:</u>

la sonde témoin est le résultat de l'hybridation de deux oligonucléotides complémentaires marqués, l'un par la biotine et l'autre par la fluorescéine.

*Principe:*

```
biotine -------------                          --------------
        -----20 bases -----FITC  +           ----X bases---
          sonde témoin                   oligonucléotide non marqué
```

*Résultats*

blanc (support luminescent + streptavidine-G6PDH) ............... 400 mV
sonde témoin non dénaturée (10 fmoles) ........................ 6927 mV
sonde témoin dénaturée et réhybridée .......................... 6695 mV
" " " " " + 100 fmoles d'oligonucléotide non marqué (20 pb) .... 865 mV
" " " " " + 100 fmoles d'oligonucléotide non marqué (64 pb) ... 1968 mV
" " " " " + 100 fmoles d'oligonucléotide non marqué (96 pb) ... 3498 mV
" " " " " + 100 fmoles d'oligonucléotide non marqué (140pb) ... 3724 mV

note : FITC = isothiocyanate de fluorescéine
pb = paires de bases

Les résultats obtenus montrent que l'utilisation d'une sonde ADN marquée sur chaque brin permet de réaliser un dosage d'un ADN homologue non marqué . Pour obtenir la meilleure sensibilité possible, il est nécessaire que l'ADN marqué (sonde) et l'ADN à doser soient de tailles identiques. L'utilisation d'oligonucléotides marqués conduit ainsi à un déplacement plus faible qu'avec des séquences obtenues par amplification.

Exemple 6 : multi-marquage spécifique de la séquence amplifiée:

L'utilisation d'oligonucléotides marqués différemment pour produire des séquences doublement marquées vient d'être décrite. Ce système se heurte à un problème de spécificité car la réaction d'amplification génère des séquences doublement marquées non spécifiques. Ces séquences sont produites par hybridations non spécifiques des oligonucléotides marqués et, pour les éliminer, il est nécessaire de réaliser une étape chromatographique ou une électrophorèse. Sans cette étape, il est possible de trouver des conditions qui diminuent la production non spécifique (température élevée, faible concentration en oligonucléotides) mais la sensibilité de la méthode reste faible et ce type de marquage n'est applicable que pour l'étude comparative permettant de détecter la présence de mutations ponctuelles ou pour réaliser un typage comme décrit à l'exemple 3, 3ème modèle ci-dessus, mais en aucun cas pour le dépistage de virus car alors le "bruit de fond" doit être le plus faible possible.

Principe du multi-marquage spécifique de la séquence amplifiée

La réaction d'amplification enzymatique (PCR) permet de produire un grand nombre de copies d'une cible donnée, cependant il est possible de produire des fragments de plus petites tailles, du fait de l'hybridation non spécifique des oligonucléotides utilisés comme amorces de la réaction . De ce fait, l'identification des produits d'amplification nécessite soit une séparation par électrophorèse, soit une hybridation spécifique avec une sonde marquée pour quantifier la quantité de cible amplifiée . Le dosage proposé utilise une base modifiée (dUTP-biotine, dATP-biotine ou DUTP-digoxigénine) à une concentration telle que, lors de la réaction d'amplification, seulement deux molécules marquées soient incorporées dans la séquence spécifique.En général, les séquences non spécifiques sont le résultat d'hybridations entre les amorces et de ce fait les séquences non spécifiques sont plus courtes que la séquence à étudier. La probabilité de double marquage de séquences non spécifiques par la base modifiée est donc relativement plus faible.

Le tableau V ci-dessous donne les valeurs théoriques du pourcentage d'incorporation de plusieurs nucléotides biotinylés (dUPT-biotine) dans des séquences d'ADN .

La séquence non spécifique est constituée des oligonucléotides amorces qui peuvent s'hybrider entre eux et permettre ainsi l'élongation d'un maximum de 20 paires de bases. La séquence spécifique, dans le cas étudié, est de 200 paires de bases. Le rapport dTTP/dUTP-biotine est égal à 50 et on considère que l'analogue biotinylé est reconnu par l'enzyme deux fois moins bien que le nucléotide naturel.

TABLEAU V

| Groupes biotine incorporés | élongation de 20 paires de bases | élongation de 200 paires de bases | marquage non spécifique |
|---|---|---|---|
| 2 | 0,420% | 18,49% | 2,2% |
| 3 | 0,012% | 6,10% | 0.19% |
| 4 | <0,001% | 1,49% | 0,013% |
| 5 | <0,001% | 0,29% | <0,001% |
| Total | 0,434% | 26,50% | 1,6% |

La valeur de multi-marquage non spécifique ainsi obtenue est de l'ordre de 1% si on considère que les quantités de séquences produites sont identiques.

Le dosage des séquences doublement marquées est réalisé par une adsorption préalable sur du Sepharose®-avidine (ou streptavidine)-luciférase-oxydoréductase (voir "préparation des immunoadsorbants bioluminescents"). Du fait de l'immobilisation de la séquence sur le Sepharose®, une seule biotine par molécule de séquence se lie à l'adsorbant . Le milieu contient du dUTP-biotine libre réagissant plus rapidement que la biotine de l'ADN immobilisé. Après réaction, un conjugué streptavidine-G6PDH réagit avec la biotine restée libre, donc la biotine portée par une séquence d'ADN déjà immobilisée . La deshydrogénase ainsi liée à l'ADN se trouve à proximité de la luciférase et, en présence de réactif de luminescence, la production de NADH par cette deshydrogénase conduit à une émission de lumière. Le NADH produit en solution est immédiatement réoxydé par le système lactate deshydrogénase-pyruvate et ne participe pas à la réaction de luminescence.

La spécificité du dosage est apportée par le multi-marquage de la séquence étudiée. L'ADN génomique est également marqué par la biotine mais la répartition est faite au hasard et en fait peu de séquences non spécifiques sont marquées plusieurs fois si on utilise de faibles concentrations de dUTP-biotine.

La détection ne nécessite aucune étape de séparation, car la biotine en excès est masquée par l'adsorbant, et peut être réalisée directement sur 0,1 à 10 µl du milieu d'amplification .

*Protocole :*

Le milieu d'amplification est obtenu comme décrit précédemment (protocole général), si ce n'est que 10 µl d'eau sont remplacés par 10 µl de dUTP-biotine 0,05 à 0,1 mM.

La détection peut être réalisée directement sur le milieu d'amplification en utilisant un volume de 2 µl ou moins de ce milieu afin d'éviter de saturer le support "Sepharose®-avidine (ou streptavidine)" qui est préparé par couplage à 1g de Sepharose® activé par le bromure de cyanogène de 3 mg d'avidine (ou de streptavidine), 5 mg de luciférase et 1 UI de FMN oxydoréductase .

1 mg de "Sepharose®-streptavidine" est incubé avec 1 µl de milieu d'amplification pendant 30 min et 1 heure avec 0,3 mUI de streptavidine-G6PDH . Après incubation, 500 µl de réactif de bioluminescence sont ajoutés (NAD $10^{-3}$ M, G6P $3.10^{-3}$ M, pyruvate $3.10^{-3}$ M, FMN $10^{-5}$ M, décanal $6.10^{-5}$ M et 30 mUI/ml de LDH dans du tampon phosphate 0,1 M). La luminescence de chaque tube est mesurée pendant 10s.

*Résultats :*

Différentes quantités d'une séquence d'ADN spécifique ont été amplifiées en présence de 1 µg d'ADN non spécifique. Le tableau VI montre les résultats obtenus avec un rapport dTTP/dUTP-biotine = 50. L'ADN non spécifique donne un "blanc" légèrement supérieur au blanc sans ADN. Toutefois, en réalisant le dosage avec 1 µl, la limite de détection obtenue après 30 cycles d'amplification est inférieure à 1000 molécules de séquence cible.

TABLEAU VI

| molécules | blanc | $10^3$ | $10^4$ | $2.10^5$ | $10^6$ | $5.10^6$ |
|---|---|---|---|---|---|---|
| Signal ( mvolts) | 190 | 400 | 550 | 800 | 990 | 1190 |

Cette technique est d'autant plus spécifique et sensible que la séquence amplifiée est plus longue. L'allongement de la séquence amplifiée, par exemple au-delà de 1000 paires de bases, ce qui est possible en utilisant les méthodes d'amplification actuellement connues, permettrait donc d'augmenter sa spécificité et sa sensibilité .

En résumé, la méthode d'analyse par bioluminescence selon l'invention permet de réaliser, sans étape de séparation, différentes analyses (dosage et/ou détection) de séquences d'acides nucléiques. Cette méthode est particulièrement adaptée à la détection de séquences produites par amplification . Des résultats quantitatifs sont obtenus par les différents procédés décrits. Ces procédés peuvent être appliqués à la détection de particules virales ou de bactéries mais aussi pour identifier des mutations ou des variations alléliques du génome humain (maladies héréditaires, oncogènes, typage HLA).

Le dosage sandwich est adapté au dosage de virus et bactéries car il présente de bonnes spécificité et sensibilité.

Le dosage par compétition ne nécessite pas de produire des ADN simples brins si la sonde est homologue à la séquence à doser.

Cette méthode est intéressante pour étudier les variations alléliques car elle permet de travailler dans des conditions d'équilibre permettant de comparer l'association de différentes sondes.

Le dosage par multimarquage est adapté à la détection de virus et bactéries, cette méthode est plus rapide que les précédentes car le marquage est réalisé lors de la réaction d'amplification. Elle nécessite cependant que la réaction d'amplification produise une séquence spécifique d'au moins 200 paires de bases. Elle ne permet pas d'étudier la nature de la séquence amplifiée et ne peut pas être utilisée pour l'identification de mutations ponctuelles.

REFERENCES BIBLIOGRAPHIQUES


1. Meinkoth, J. et coll., Anal. Biochem. 138 (1984) 267-284.
2. Spector, S.A. et coll., Clin. Chem. 31(9) (1985) 1514-1520.
3. Pereira, H.G. et coll., BioEssays 4 (1986) 110-113.
4. Leary, J. et coll., Proc, Natl. Acad. Sci. 80 (1983) 4045-4049.
5. Jablonski E. coll., Nucleic Acids Research 14 (1986) 6115-6128.
6. Wong, C. et coll., Nature 330 (1987) 384-386.
7. Ehrlich, H.A. et coll., Nature 331 (1988) 461-462.
8. Demande de brevet WO 83/01459 (ORION-YHTYM'A' OY).
9. Demande de brevet EP 0 139 489 (ORTHO DIAGNOSTIC SYSTEMS).
10. Demande de brevet EP 0 238 332 (CETUS CORPORATION).
11. Farghaly A.H., J. Cell. Comp. Physiol. 36 (1969) 165-184.
12. Gunsalus-Miguel A. et coll., J. Biol. Chem. 247 (1972) 398-404.
13. Baldwin T.O. et coll. J. Biol. Chem. 250 (1975), 2763-2768.
14. Garbutt G.J. et coll., Clin. Chem. 31 (1985) 1203-1206.
15. Forster A.C. et coll., Nucleic Acids Research, 13 (1985) 745-761.
16. Lebacq P. et coll., J. Biochem. Biophys. Methods, 15 (1988) 255-266.
17. Tchen P. et coll., Proc. Natl. Acad. Sci. USA, 81 (1984) 3466-3470.
18. Kumar A. et coll., Anal. Biochem. 169 (1988) 376-382.
19. Cannolly B.A. (The synthesis of oligonucleotides containing a primary amino group at the 5' terminus) Nucl. Acids. Res. 15. (1987).
20. Agrawal S. et coll., Nucl. Acids Res. 14 (1986) 6227-6245.
21. Todd J.A., Nature, 329 (1987) 599-604.


## Revendications

1. Procédé d'analyse d'une séquence spécifique d'ADN ou d'ARN, caractérisé en ce qu'il comprend les étapes consistant, en utilisant :

   a) un support présentant une surface poreuse et/ou fibreuse sur laquelle sont immobilisés d'une part, les enzymes luciférase et oxydoréductase d'un système de bioluminescence et d'autre part, un substituant capable de fixer, directement ou indirectement, par l'intermédiaire d'au moins une réaction d'affinité ou par hybridation spécifique, une séquence nucléotidique,

   b) un conjugué d'enzyme comprenant une enzyme capable de produire un composé nucléotide intermédiaire, utilisable par l'oxydoréductase du système de bioluminescence, pour conduire à l'émission d'un signal de bioluminescence et d'une molécule capable de se lier par affinité avec un marqueur de séquence nucléotidique ou de s'hybrider avec une telle séquence, et

   c) une solution aqueuse comprenant les substrats de l'enzyme du conjugué, les constituants du système de bioluminescence autres que les enzymes et le composé nucléotide intermédiaire, ainsi que les constituants d'un système de destruction du composé nucléotide intermédiaire produit par l'enzyme dudit conjugué non reliée au support, à

   -1° selon une première étape comportant trois modes de réalisation :

   - soit mettre un échantillon contenant l'ADN ou l'ARN à analyser, éventuellement amplifié, au moins doublement marqué par marquage chimique ou enzymatique avant l'analyse, par marquage synthétique au cours de l'amplification ou par hybridation avec une ou plusieurs sondes spécifiques, en contact, simultanément ou successivement, avec un support a) et un conjugué b) appropriés,

- soit mettre un échantillon contenant l'ADN ou l'ARN à analyser, de préférence amplifié, non marqué, en présence de séquences homologues d'ADN ou d'ARN, de préférence amplifiées, doublement marquées et soumettre l'ensemble à dénaturation puis à réhybridation, et mettre le mélange obtenu en contact, simultanément ou successivement, avec un support a) et un conjugué b) appropriés,
- soit mettre en contact, simultanément ou successivement, d'une part un échantillon contenant l'ADN ou l'ARN à analyser, de préférence amplifié, avec un support a) portant une séquence nucléotidique complémentaire d'une partie dudit ADN ou ARN, cette séquence pouvant être fixée sur le support au cours de l'analyse, par réaction d'affinité spécifique entre un substituant dudit suport et un substituant porté par un oligonucléotide présentant une affinité spécifique pour ledit substituant du support et, d'autre part un conjugué d'enzyme b) dans lequel l'enzyme est conjuguée à une séquence nucléotidique complémentaire d'une autre partie dudit ADN ou ARN et non complémentaire de la séquence nucléotidique portée par le support,

-2° mettre l'ensemble obtenu sous 1° en présence d'un excès de la solution c) ;
-3° laisser reposer jusqu'à obtention d'un signal stable; puis
-4° mesurer la lumière émise; et éventuellement
-5° déterminer, à l'aide d'un système d'étalonnage, la quantité de séquence à doser dans l'échantillon étudié, à partir de la mesure effectuée sous 4°.

**2.** Procédé selon la revendication 1, caractérisé en ce que le support a) est constitué d'un polymère osidique tel que l'agarose ou un agarose modifié, d'acrylamide, d'un copolymère acrylique, de verre poreux ou d'une matière fibreuse.

**3.** Procédé selon la revendication 1 ou 2, caractérisé en ce que
- l'enzyme du conjugué b) est la glucose-6-phosphate deshydrogénase ;
- le composé nucléotide intermédiaire est le NADH ;
- la luciférase est la luciférase de B. Harveyi qui est fixée sur le support a), notamment à base d'agarose, en présence de l'oxydoréductase correspondante; et
- la solution c) comprend, en tant que réactifs, du glucose-6-phosphate, du NAD, du FMN, un aldéhyde à longue chaîne tel que le décanal, de la LDH et du pyruvate.

**4.** Procédé selon l'une des revendications 1 à 3, caractérisé en ce qu'il utilise une réaction de type "sandwich" et en ce qu'à l'étape 1°, la séquence à analyser est substituée doublement, éventuellement au cours de l'analyse, par un premier substituant capable de se lier, directement ou indirectement, au support a) par une réaction d'affinité, et par un second substituant capable de se lier au conjugué b) par une réaction d'affinité.

**5.** Procédé selon l'une des revendications 1 à 4, caractérisé en ce qu'il utilise une réaction de type "sandwich" et en ce qu'il comprend, avant la mise en contact de l'hybride avec le support a), le conjugué b) et les réactifs de bioluminescence, une série de cycles de dénaturation et d'amplification de l'ADN ou de l'ARN, au moyen d'une polymérase.

**6.** Procédé selon l'une des revendications 1 à 3, caractérisé en ce qu'il utilise une réaction d'hybridation de type "sandwich"et en ce que le support a) porte une séquence nucléotidique capable de s'hybrider avec la séquence à analyser, complémentaire d'une partie de cette séquence et le conjugué b) est constitué d'une enzyme, notamment une deshydrogénase, et d'une séquence nucléotidique, non complémentaire de la séquence portée par le support a) et complémentaire d'une partie de la séquence à analyser, de préférence amplifiée.

**7.** Procédé selon l'une des revendications 1 à 3, caractérisé en ce qu'il utilise une réaction d'hybridation de type "sandwich" et en ce que la séquence à analyser, de préférence amplifiée, est marquée par un substituant présentant une affinité spécifique pour le substituant porté par le support a), et le conjugué b) est constitué d'une enzyme, notamment une deshydrogénase, et d'une séquence nucléotidique complémentaire d'une partie de la séquence à analyser.

**8.** Procédé selon l'une des revendications 1 à 3, caractérisé en ce qu'il fait intervenir une réaction de type "sandwich" et en ce que la séquence à analyser est multimarquée, de préférence par amplification, le support a) porte des substituants présentant une affinité spécifique pour le marqueur de ladite séquence et le conjugué b) comporte également un substituant présentant une affinité spécifique pour le marqueur

de ladite séquence.

9. Procédé selon l'une des revendications 1 à 3, caractérisé en ce que l'étape 1° fait intervenir une réaction de compétition utilisant une séquence doublement marquée par des substituants différents ou deux oligonucléotides complémentaires marqués par des substituants différents, lesdits substituants différents présentant l'un, une affinité spécifique pour le substituant spécifique porté par le support a) et l'autre, une affinité spécifique pour la molécule spécifique du conjugué b).

10. Réactif pour la mise en oeuvre du procédé selon l'une des revendications 1 à 9, caractérisé en ce qu'il se présente sous la forme d'un support présentant une surface poreuse et/ou fibreuse sur laquelle sont immobilisés d'une part, les enzymes luciférase et oxydoréductase d'un système de bioluminescence et d'autre part, un substituant capable de fixer, directement ou indirectement, par l'intermédiaire d'au moins une réaction d'affinité ou par hybridation spécifique, une séquence nucléotidique.

11. Réactif selon la revendication 10, caractérisé en ce qu'il est constitué de microbilles d'agarose modifié portant, fixés à leur surface sur les mêmes microbilles ou sur des microbilles différentes, un anticorps anti-y-globulines, une protéine A, de l'avidine ou une sonde nucléique, une luciférase d'origine bactérienne et une oxydoréductase.

12. Nécessaire pour la mise en oeuvre du procédé selon l'une des revendications 1 à 9, comprenant :
   - le réactif selon la revendication 10 ou 11, à l'abri de la lumière, et
   - un flacon contenant un conjugué de deshydrogénase et d'une molécule capable de se lier sélectivement, par affinité, avec un marqueur de séquence nucléotidique ou de s'hybrider avec une telle séquence.

13. Nécessaire selon la revendication 12, caractérisé en ce qu'il comprend :
   - un flacon contenant, à l'abri de la lumière, des microbilles d'agarose modifié, en suspension dans l'eau, ou sous forme de comprimé délitable dans l'eau, munies à leur surface d'un anticorps anti-$\gamma$-globulines ou d'une protéine A, d'une luciférase d'origine bactérienne et d'une oxydoréductase,
   - un flacon contenant un conjugué de deshydrogénase et d'une molécule capable de se lier sélectivement, par affinité, à un marqueur de séquence nucléotidique ou de s'hybrider avec une telle séquence,
   - un flacon contenant un anticorps spécifique d'un autre marqueur de séquence nucléotidique, et
   - du tampon, du NAD ou du NADP, du FMN, un aldéhyde à longue chaîne, un système capable de détruire le NADH ou le NADPH en solution, ainsi que le substrat de la deshydrogénase.

14. Nécessaire selon la revendication 12, caractérisé en ce qu'il comprend :
   - un flacon contenant, à l'abri de la lumière, des microbilles d'agarose modifié, en suspension dans l'eau, ou sous forme de comprimé délitable dans l'eau, munies à leur surface d'avidine ou de streptavidine, d'une luciférase d'origine bactérienne et d'une oxydoréductase,
   - un flacon contenant un conjugué de deshydrogénase et d'une molécule capable de se lier sélectivement, par affinité, à un marqueur de séquence nucléotidique ou de s'hybrider avec une telle séquence, et
   - du tampon, du NAD ou du NADP, du FMN, un aldéhyde à long chaîne, un système capable de détruire le NADH ou le NADPH en solution, ainsi que le substrat de la deshydrogénase.

## Patentansprüche

1. Verfahren zur Analyse einer spezifischen DNA- oder RNA-Sequenz, dadurch gekennzeichnet, daß es unter Verwendung

   a) eines Trägers, welcher eine poröse und/oder faserige Oberfläche aufweist, auf der zum einen die Enzyme Luciferase und Oxidoreduktase eines Biolumineszenzsystems und zum anderen ein Substituent, der vermittels mindestens einer Affinitätsreaktion oder über eine spezifische Hybridisierung zur direkter oder indirekter Fixierung einer Nucleotidsequenz befähigt ist, immobilisiert sind,

   b) eines Konjugats aus einem Enzym, das ein zur Produktion einer von der Oxidoreduktase des Biolumineszenzsystems verwendbaren Nucleotid-Zwischenverbindung befähigtes Enzym enthält, um die Emission eines Biolumineszenzsignals zu bewirken und aus einem Molekül, das zur affinen Bindung mit einem Nucleotidsequenzmarker oder zur Hybridisierung mit einer solchen Sequenz befähigt ist, und

c) einer wässrigen Lösung mit den Substraten des Enzyms des Konjugats, den Bestandteilen des Biolumineszenzsystems mit Ausnahme der Enzyme und der Nucleotid-Zwischenverbindung, ebenso wie den Bestandteilen eines Abbausystems für die von dem nicht an den Träger gebundenen Enzym dieses Konjugats gebildete Nucleotid-Zwischenverbindung,
die Schritte umfaßt, bestehend aus
-1. Einem ersten Schritt in drei Ausführungsformen:
- Entweder in Kontaktbringen mit einer die zu analysierenden DNA oder RNA enthaltenden Probe gleichzeitig oder nacheinander mit einem geeigneten Träger a) und einem geeigneten Konjugat b), wobei die Probe gegebenenfalls amplifiziert ist, zumindest aber doppelt markiert mittels chemischer oder enzymatischer Markierung vor der Analyse, mittels synthetischer Markierung im Verlaufe der Amplifikation oder mittels Hybridisierung mit einer oder mehreren spezifischen Sonden,
- oder Zusammenbringen einer die zu analysierende DNA oder RNA enthaltenden, vorzugsweise amplifizierten, nicht markierten probe mit homologen, vorzugsweise amplifizierten, doppelt markierten DNA- oder RNA-Sequenzen, das ganze einer Denaturierung unterziehen und darauf einer Rehybridisierung, und in Kontaktbringen der erhaltenen Mischung, gleichzeitig oder nacheinerander, mit einem geeigneten Träger a) und einem geeigneten Konjugat b),
- oder in Kontaktbringen, gleichzeitig oder nacheinander, einerseits einer die zu analysierende, vorzugsweise amplifizierte DNA oder RNA enthaltenden Probe mit einem Träger a), welcher eine zu einem Abschnitt dieser DNA oder RNA komplementäre Nucleotidsequenz trägt, wobei diese Sequenz während der Analyse über eine spezifische Affinitätsreaktion zwischen einem Substituenten dieses Trägers und einem auf einem Oligonucleotid sitzenden Substituenten mit spezifischer Affinität zu diesem Trägersubstituenten an den Träger fixiert werden kann, und andererseits ein Enzymkonjugat b), in welchem das Enzym mit einer Nucleotidsequenz konjugiert ist, welche zu einem anderen Abschnitt der DNA oder RNA komplementär und zu der auf dem Träger sitzenden Nucleotidsequenz nicht komplementär ist;

-2. Zusammenbringen des unter 1. erhaltenen Komplexes mit einem Überschuß von Lösung c);
-3. Ruhenlassen bis zum Erhalt eines stabilen Signals; sodann
-4. Messen des ausgesendeten Lichts und gegebenenfalls
-5. Bestimmen der in die untersuchte Probe gemäß der Messung nach 4. zu dosierenden Sequenzmenge mit Hilfe eines Eichsystems.

**2.** Verfahren nach Anspruch 1, dadurch gekennzeichnet, daß der Träger a) aus einem osidischen polymer wie Agarose oder modifizierter Agarose, aus Acrylamid, aus einem Acrylcopolymeren, aus porösem Glas oder einem faserigen Material aufgebaut ist.

**3.** Verfahren nach den Ansprüchen 1 or 2, dadurch gekennzeichnet, daß
- das Enzym des Konjugats b) Glukose-6-phosphat-Dehydrogenase ist;
- die Nucleotid-Zwischenverbindung NADH ist;
- die Luciferase eine Luciferase aus B. Harveyi ist, welche auf dem Träger a), insbesondere auf Basis von Agarose, in Gegenwart der entsprechenden Oxidoreduktase fixiert ist; und
- die Lösung c) als Reagenzien Glucose-6-phosphat, NAD, FMN, einen langkettigen Aldehyd wie Dekanal, LDH und Pyruvat enthält.

**4.** Verfahren nach jedem der Ansprüche 1-3, dadurch gekennzeichnet, daß eine "Sandwich"- Reaktion angewandt wird und daß im ersten Schritt die zu analysierende Sequenz zweifach substituiert wird, gegebenenfalls im Verlauf der Analyse, und zwar mittels eines ersten Substituenten, welcher befähigt ist, sich direkt oder indirekt an den Träger a) mittels einer Affinitätsreaktion anzuheften und mittels eines zweiten Substituenten, welcher befähigt ist, sich an das Konjugat b) mittels einer Affinitätsreaktion zu binden.

**5.** Verfahren nach jedem der Ansprüche 1-4, dadurch gekennzeichnet, daß eine "Sandwich"- Reaktion angewandt wird, und dadurch daß, bevor das Hybrid mit dem Träger a), dem Konjugat b) und den Biolumineszenz-Reagenzien in Kontakt gebracht wird, mittels einer Polymerase eine Reihe von Denaturierungs und Amplifikationszyklen mit der DNA oder RNA durchgeführt wird.

**6.** Verfahren nach jedem der Ansprüche 1-3, dadurch gekennzeichnet, daß für die Hybridisierungsreaktion eine "Sandwich"-Reaktion angewandt wird und dadurch, daß der Träger a) eine Nucleotidsequenz enthält, die befähigt ist, mit der zu analysierenden, mit einem Abschnitt dieser Sequenz komplementären Sequenz

eine Hybridisierung einzugehen und daß sich das Konjugat b) aus einem Enzym, insbesondere einer Dehydrogenase und einer Nucleotidsequenz, vorzugsweise amplifiziert, zusammensetzt, welche nicht komplementär zu der auf dem Träger a) enthaltenen Sequenz und komplementär zu einem Abschnitt der zu analysierenden Sequenz ist.

**7.** Verfahren nach jedem der Ansprüche 1-3, dadurch gekennzeichnet, daß für die Hybridisierungsreaktion eine "Sandwich"-Reaktion angewandt wird und dadurch, daß die zu analysierende, vorzugsweise amplifizierte Sequenz mit einem Substituenten markiert ist, welcher eine spezifische Affinität zu dem auf dem Träger a) enthaltenen Substituenten aufweist, und daß das Konjugat b) aus einem Enzym, insbesondere einer Dehydrogenase, und einer Nucleotidsequenz besteht, welche komplementär zu einem Abschnitt der zu analysierenden Sequenz ist.

**8.** Verfahren nach jedem der Ansprüche 1-3, dadurch gekennzeichnet, daß eine "Sandwich"- Reaktion angewandt wird und darduch, daß die zu analysierende Sequenz vielfach markiert ist, vorzugsweise mittels Amplifikation, daß der Träger a) Substituenten enthält, welche eine spezifische Affinität zum Marker dieser Sequenz aufweisen und daß das Konjugat b) ebenfalls einen Substituenten enthält, welcher eine spezifische Aktivität zum Marker dieser Sequenz aufweist.

**9.** Verfahren nach jedem der Ansprüche 1-3, dadurch gekennzeichnet, daß man in Schritt 1 kompetitiv reagieren läßt, indem man eine mit unterschiedlichen Substituenten zweifach markierte Sequenz verwendet oder zwei komplementäre Oligonucleotide, welche mit unterschiedlichen Substituenten markiert sind, wobei diese unterschiedlichen Substituenten zum einen eine spezifische Affinität zu dem auf dem Träger a) befindlichen spezifischen Substituenten und zum anderen eine spezifische Affinität zu dem spezifischer. Molekül des Konjugats b)aufweisen.

**10.** Reagenz zur Durchführung des Verfahrens nach jedem der Ansprüche 1-9, dadurch gekennzeichnet, daß es in Form eines Trägers ausgebildet ist, welcher eine poröse und/oder faserige Oberfläche aufweist, auf welcher einesteils die Enzyme Luciferase und Oxidoreduktase eines Biolumineszenzsystems immobilisiert sind und anderenteils ein Substituent, welcher in der Lage ist, mit Hilfe mindestens einer Affinitätsreaktion oder mittels spezifischer Hybridisierung direkt oder indirekt eine Nucleotidsequenz zu fixieren.

**11.** Reagenz nach Anspruch 10, dadurch gekennzeichnet, daß es aus Mikrokügelchen aus modifizierter Agarose besteht, wobei an die Oberflächen auf gleichen oder unterschiedlichen Mikrokügelchen ein Anti-$\gamma$-globulin-Antikörper, ein Protein A, Avidin oder eine Nucleinsäuresonde, eine Luciferase bakteriellen Ursprungs und eine Oxidoreduktase fixiert sind.

**12.** Kit zur Durchführung des Verfahrens nach jedem der Ansprüche 1-9, umfassend
- das Reagenz nach den Ansprüchen 10 oder 11, geschützt gegen Licht, und
- ein Fläschchen mit einem Konjugat aus Dehydrogenase und einem Molekül, welches befähigt ist, sich über seine Affinität selektiv an einem Nucleotidsequenzmarker zu binden oder eine Hybridisierung mit einer solchen Sequenz einzugehen.

**13.** Kit nach Anspruch 12, dadurch gekennzeichnet, daß es enthält:
- ein Fläschchen, welches gegen Licht geschützt Mikrokügelchen aus modifizierter Agarose in wässriger Suspension oder in Form von in Wasser löslichen Tabletten enthält, welche auf ihrer Oberfläche mit einem Anti-$\gamma$-globulin-Antikörper oder einem Protein A, einer Luciferase bakteriellen Ursprungs und einer Oxidoreduktase behaftet sind,
- ein Fläschchen mit einem Konjugat aus Dehydrogenase und einem Molekül, welches befähigt ist, sich über seine Affinität selektiv an einen Nucleotidsequenzmarker zu binden oder eine Hybridisierung mit einer solchen Sequenz einzugehen,
- ein Fläschchen mit einem zu einem anderen Nucleotidsequenzmarker spezifischen Antikörper, und
- einen Puffer, NAD oder NADP, FMN, einen langkettigen Aldehyd, ein zur Zerstörung der NADH oder NADPH in Lösung befähigtes System sowie das Substrat der Dehydrogenase.

**14.** Kit nach Anspruch 12, dadurch gekennzeichnet, daß es enthält:
- ein Fläschchen, welches gegen Licht geschützt Mikrokügelchen aus modifizierter Agarose in wässriger Suspension oder in Form von in Wasser löslichen Tabletten enthält, welche auf ihrer Oberfläche Avidin oder Streptavidin, eine Luciferase bakteriellen Ursprungs und eine Oxidoreduktase enthalten,
- ein Fläschchen mit einem Konjugat aus Dehydrogenase und einem Molekül, welches befähigt ist, sich

über seine Affinität selektiv an einen Nucleotidsequenzmarker zu binden oder eine Hybridisierung mit einer solchen Sequenz einzugehen, und
- einen Puffer, NAD oder NADP, FMN, einen langkettigen Aldehyd, ein zur Zerstörung der NADH oder NADPH in Lösung befähigtes System sowie das Substrat der Dehydrogenase.

## Claims

1. Process for the analysis of a specific sequence of DNA or RNA, characterized in that it comprises stages which consist of, using:
   a) a support having a porous and/or fibrous surface on which are fixed on the one hand, the luciferase and oxidoreductase enzymes of a bioluminescence system, and on the other hand, a substituent capable of fixing, directly or indirectly, by means of at least one affinity reaction or by specific hybridization, a nucleotide sequence,
   b) an enzyme conjugate containing an enzyme capable of producing an intermediate nucleotide compound, which can be used by the oxidoreductase of the bioluminescence system, to produce the emission of a bioluminescence signal and of a molecule capable of linking itself by affinity with a nucleotide sequence marker or of hybridizing with such a sequence, and
   c) an aqueous solution containing the substrates of the enzyme of the conjugate, the constituents of the bioluminescence system other than the enzymes and the intermediate nucleotide compound, as well as the constituents of a destruction system of the intermediate nucleotide compound produced by the enzyme of the said conjugate not linked to the support,
   -1° according to a first stage comprising three embodiments:
   - either putting a sample containing DNA or RNA to be analyzed, optionally amplified, labelled at least twice by chemical or enzymatic labelling before the analysis, by synthetic labelling during amplification or by hybridization with one or more specific probes, in simultaneous or successive contact with an appropriate support a) and conjugate b),
   - or putting a sample containing DNA or RNA to be analyzed, preferably amplified, and non-labelled, in the presence of homologous DNA or RNA sequences, preferably amplified, labelled twice and of subjecting the whole to denaturing then rehybridization, and putting the mixture obtained in simultaneous or successive contact with an appropriate support a) and conjugate b),
   - or putting in simultaneous or successive contact, on the one hand a sample containing the DNA or RNA to be analyzed, preferably amplified, with a support a) carrying a nucleotide sequence complementary to a part of the said DNA or RNA, this sequence being able to be fixed on the support during analysis, by specific affinity reaction between a substituent of the said support and a substituent carried by an oligonucleotide having a specific affinity for the said substituent of the support and, on the other hand an enzyme conjugate b) in which the enzyme is conjugated to a nucleotide sequence complementary to another part of the said DNA or RNA and not complementary to the nucleotide sequence carried by the support;

   -2° putting the whole mixture obtained in 1° in the presence of an excess amount of solution c);
   -3° leaving at rest until a stable signal is obtained; then
   -4° measuring the light emitted; and optionally
   -5° determining, using a calibration system, the quantity of sequence to be analyzed in the sample studied, from the measurement carried out in 4°.

2. Process according to claim 1, characterized in that the support a) is constituted by an oside polymer such as agarose or a modified agarose, acrylamide, an acrylic copolymer, porous glass or a fibrous material.

3. Process according to claim 1 or 2, characterized in that:
   - the enzyme of conjugate b) is glucose-6-phosphate dehydrogenase;
   - the intermediate nucleotide compound is NADH;
   - the luciferase is B. Harveyi luciferase which is fixed on support a), notably based on agarose, in the presence of the corresponding oxidoreductase; and
   - solution c) contains, as reagents, glucose-6-phosphate , NAD, FMN, a long-chain aldehyde such as decanal, LDH and pyruvate.

4. Process according to one of claims 1 to 3, characterized in that it uses a "sandwich" type reaction and in that in stage 1°, the sequence to be analyzed is substituted twice, optionally during the analysis, by a

first substituent capable of being linked, directly or indirectly, to support a) by an affinity reaction, and by a second substituent capable of being linked to conjugate b) by an affinity reaction.

5. Process according to one of claims 1 to 4, characterized in that it uses a "sandwich" type reaction and in that it comprises, before the hybrid is put in contact with support a), conjugate b) and the bioluminescence reagents, a series of denaturing and amplification cycles of the DNA or RNA, by means of a polymerase.

6. Process according to one of claims 1 to 3, characterized in that it uses a "sandwich" type hybridization reaction and in that the support a) carries a nucleotide sequence capable of hybridizing with the sequence to be analyzed, complementary to a part of this sequence, and conjugate b) is constituted by an enzyme, in particular a dehydrogenase, and a nucleotide sequence, not complementary to the sequence carried by support a) and complementary to a part of the sequence to be analyzed, preferably amplified.

7. Process according to one of claims 1 to 3, characterized in that it uses a "sandwich" type hybridization reaction and in that the sequence to be analyzed, preferably amplified, is labelled by a substituent having a specific affinity for the substituent carried by support a), and conjugate b) is constituted by an enzyme, in particular a dehydrogenase, and a nucleotide sequence complementary to a part of the sequence to be analyzed.

8. Process according to one of claims 1 to 3, characterized in that it uses a "sandwich" type reaction and in that the sequence to be analyzed is multi-labelled, preferably by amplification, support a) carries substituents having a specific affinity for the label of the said sequence and conjugate b) also contains a substituent having a specific affinity for the marker of the said sequence.

9. Process according to one of claims 1 to 3, characterized in that stage 1° uses a competition reaction using a sequence labelled twice with different substituents or two complementary oligonucleotides labelled by different substituents, one of the said different substituents having a specific affinity for the specific substituent carried by support a) and the other having a specific affinity for the specific molecule of conjugate b).

10. Reagent for the implementation of the process according to one of claims 1 to 9, characterized in that it is in the form of a support having a porous and/or fibrous surface on which are fixed on the one hand the luciferase and oxidoreductase enzymes of a bioluminescence system and on the other hand a substituent capable of fixing, directly or indirectly, by means of at least one affinity reaction or by specific hybridization, a nucleotide sequence.

11. Reagent according to claim 10, characterized in that it is constituted by microbeads of modified agarose carrying, fixed to their surface on the same microbeads on different microbeads an anti-γ-globulin antibody, a protein A, avidin or a nucleic probe, a luciferase of bacterial origin and an oxidoreductase.

12. Kit for the implementation of the process according to one of claims 1 to 9, comprising:
   - the reagent according to claim 10 or 11, protected from light, and
   - a flask containing a conjugate of dehydrogenase and of a molecule capable of being linked selectively, by affinity, with a nucleotide sequence marker, or of hybridizing with such a sequence.

13. Kit according to claim 12, characterized in that it comprises:
   - a flask containing, protected from light, microbeads of modified agarose, in suspension in water, or in the form of a tablet which breaks down in water, provided on their surface with an anti-γ-globulin antibody or a protein A, a luciferase of bacterial origin and an oxidoreductase,
   - a flask containing a conjugate of dehydrogenase and a molecule capable of being linked selectively, by affinity, to a nucleotide sequence marker or of hybridizing with such a sequence,
   - a flask containing a specific antibody of another nucleotide sequence marker, and
   - a buffer solution, NAD or NADP, FMN, a long-chain aldehyde, a system capable of destroying the NADH or the NADPH in solution, as well as the substrate of the dehydrogenase.

14. Kit according to claim 12, characterized in that it comprises:
   - a flask containing, protected from light, microbeads of modified agarose, in suspension in water, or in the form of a tablet which breaks down in water, provided on their surface with avidin or streptavidin, a luciferase of bacterial origin and an oxidoreductase,

- a flask containing a conjugate of dehydrogenase and a molecule capable of being linked selectively, by affinity, to a nucleotide sequence marker or of hybridizing with such a sequence, and
- a buffer solution, NAD or NADP, FMN, a long-chain aldehyde, a system capable of destroying the NADH or NADPH in solution, as well as the substrate of the dehydrogenase.